# EUROPÄISCHE PATENTSCHRIFT

(11) **EP 0 647 848 B1**
(45) Veröffentlichungstag und Bekanntmachung des Hinweises auf die Patenterteilung: **29.12.1999**
(21) Anmeldenummer: 94115350.4
(22) Anmeldetag: 29.09.1994
(51) Int. Cl.: G01N 33/18, G01N 27/06, G01N 27/02

(54) **Verfahren zum Überwachen von Wasser auf organische Verunreinigungen**
Method for monitoring organic contamination in water
Méthode pour le contrôle de contaminations organiques dans l'eau

(30) Priorität: 08.10.1993 DE 4334434
(43) Veröffentlichungstag der Anmeldung: 12.04.1995
(73) Patentinhaber: HOECHST AKTIENGESELLSCHAFT, 65929 Frankfurt am Main (DE)
(72) Erfinder: Schunck, Günter, Dr., LFE, D-63477 Maintal (DE); Wasel-Nielen, Joachim, Dr., D-60325 Frankfurt (DE); Lauer, Christian, Dr., D-65929 Frankfurt (DE); Melzer, Werner, Prof. Dr., D-65835 Liederbach (DE)

(56) Entgegenhaltungen:
- EP-A- 0 433 499
- GB-A- 2 029 014
- US-A- 3 635 564
- US-A- 4 801 551

## Beschreibung

Die Erfindung betrifft ein Verfahren zum Überwachen von Wasser, insbesondere von Kesselspeisewasser auf organische Verunreinigungen durch Messen der Leitfähigkeit.

Die Erfindung betrifft ferner eine Vorrichtung zum Durchführen des Verfahrens.

In Kesselspeisewasser müssen Verunreinigungen, insbesondere gelöste halogenierte Kohlenwasserstoffe vermieden werden, da sie Korrosionen am Dampfkessel verursachen. Zum Nachweis gelöster organischer Verunreinigungen ist das DOC (Dissolved Organic Carbon)-Verfahren bekannt. Nachteilig bei diesem Verfahren ist, daß flüchtige Organika und Hetero-Atome, z. B. Säurebildner, wie Chlor- und Schwefelverbindungen, nicht erfaßt werden (vgl. dazu "Kontinuierliche TOC-Meßtechnik in der Abwasserüberwachung" atp-Automatisierungstechnische Praxis 34 (1992)3.)

Es ist ferner aus der EP 0 150 923 B1 ein Verfahren bekannt, bei dem die im Kessel stattfindende thermische Umwandlung in einem UV-Reaktor nachgeahmt werden soll. Dabei wird die Leitfähigkeit des Wassers nach dem Bestrahlen gemessen. Nachteilig bei diesem Verfahren ist, daß durch die UV-Oxidation die Umwandlung von z. B. halogenierten Kohlenwasserstoffen in lösliche, dissoziierbare Komponenten nicht in gewünschtem Maße stattfindet.

Ferner ist aus der EP 0 433499 A1 ein Verfahren bekannt, bei der eine Probe Wasser bei weniger als 450 °C verdampft und kondensiert wird, bevor die Impedanz des Kondensats gemessen wird.

Die US 3,635,564 beschreibt ein Verfahren, um indirekt aus nur einem Strom die löslichen organischen Substanzen ohne Verdampfung oder thermische Behandlung über die Messung von Refraktivität und Leitfähigkeit zu bestimmen.

Weiter beschreibt US 4,801,551 ein Verfahren, um den Gehalt an gelöstem Kohlendioxid/Bicarbonat in einem Wasserstrom zu bestimmen, in dem die Leitfähigkeit vor und nach Erwärmen von 25 °C auf 95 °C gemessen wird.

Vor diesem Stand der Technik lag der Erfindung die Aufgabe zugrunde, ein alternatives Verfahren zum Überwachen von Wasser zu entwickeln.

Diese Aufgabe wird durch ein Verfahren der eingangs genannten Art gelöst, das dadurch gekennzeichnet ist, daß man von einem Probenwasserstrom einen Teilstrom abzweigt und die Leitfähigkeit mißt, den anderen Teilstrom bei 300-500 °C verdampft, den Dampf thermisch bei Temperaturen von 800-1000 °C behandelt, den behandelten Dampf kondensiert, die Leitfähigkeit des Kondensats mißt und die Differenz der gemessenen Leitfähigkeiten bildet.

Das Verfahren läßt sich noch verbessern, wenn nach dem Kondensieren des Dampfes vorhandenes Gas abgetrennt und Kondensat und Gas getrennt gefördert werden. Ferner kann die Probe vor dem Teilen durch einen lonenaustauscher geleitet werden.

Zur Durchführung des erfindungsgemäßen Verfahrens eignet sich eine Vorrichtung, die dadurch gekennzeichnet ist, daß einem Probenwasserstromteiler zwei Leitfähigkeitsmeßzellen nachgeschaltet sind, wobei zwischen Probenwasserstromteiler und einer der Leitfähigkeitsmeßzellen in Richtung Meßzelle ein Verdampfer, ein Reaktor und ein Kondensator angeordnet sind und die Meßzellen mit einer elektronischen Meßwertverarbeitungseinrichtung verbunden sind.

Dem Kondensator kann ein Gasabscheider nachgeschaltet, dem Verdampfer eine Dampfsperre und dem Probenteiler ein lonenaustauscher vorgeschaltet sein. Den Meßzellen können Fördereinrichtungen für die Probenwasserströme nachgeschaltet sein.

Die Vorteile des Verfahrens sind im wesentlichen darin zu sehen, daß das thermische Verfahren identisch mit den Vorgängen im Dampfkessel ist und damit eine direkte Aussage über das Maß einer schädigenden Wirkung liefert.

Im folgenden wird die Erfindung anhand eines Flußdiagramms (Fig.) näher erläutert. Ein Probenwasserstrom (Leitung 3) wird nach Passieren eines lonenaustauschers (1) in einem Probenwasserstromteiler (2) in zwei Probenwasserteilströme (Leitungen 4 und 5) aufgeteilt. Der erste Teilstrom (Leitung 4) wird mittels einer Pumpe (7) dem Probenwasserstromteiler (2) entnommen und einer gegebenenfalls thermostatisierten Leitfähigkeitsmeßzelle (6) zugeführt, wo die elektrische Leitfähigkeit gemessen wird. Der zweite Probenwasserteilstrom (Leitung 5) wird mittels Pumpe (11) dem Probenwasserstromteiler (2) entnommen und einem Verdampfer (8) zugeführt, wo er bei 300-500° C verdampft wird. Der Dampf wird anschließend in einem Reaktor (9) thermisch bei Temperaturen von 800-1000° C und nahezu Atmosphärendruck behandelt, wobei sich die organischen Bestandteile zersetzen. Der behandelte Dampf wird in einem nachgeschalteten Kondensator (10) kondensiert, wobei die zersetzten Bestandteile, im wesentlichen Säuren, dissoziieren und zu einer Leitfähgikeitserhöhung führen. Das Kondensat wird, gegebenenfalls nach Abtrennung von Gas in Gasabscheider (12), einer gegebenenfalls thermostatisierten Leitfähigkeitsmeßzelle (13) zugeführt. Die in den Meßzellen (6) und (13) gemessenen Leitfähigkeiten werden in einer Meßwertverarbeitungseinrichtung (14) verarbeitet. Entweder wird das Meßsignal der Leitfähigkeitszelle hinter dem Reaktor als Maß für die Verunreinigung verwendet oder - bei gegebenenfalls schwankender Grundleitfähigkeit - wird die Differenz aus beiden Leitfähigkeitswerten als Maß für die Verunreinigung, insbesondere halogenierter organischer Verbindungen, benutzt. Eventuell abgeschiedenes Gas wird dem Gasabscheider (12) über Leitung (15) mittels Pumpe (16) entnommen. Es kann zweckmäßig sein, dem Verdampfer (8) eine Dampfsperre (17) vorzuschalten. Pumpe (18), Leitung (19) und Reservoir (20) sollen einen Kühlmittelkreislauf andeuten, die Bezugsziffern (21) und (22) jeweils auf die Energiezufuhreinrichtung für Verdampfer (8) und Reaktor (9) hinweisen. Das getrennte Absaugen von Gas und Kondensat aus dem Gasabscheider (12) in Verbindung mit der vorgeschalteten Dampfsperre (17) bewirkt eine Dämpfung der durch das Verdampfen des Probenwassers auftretenden Druckstöße, die zu starken Schwankungen des Durchflusses führen können und damit die durchflußabhänigige Leitfähigkeitsmessung beeinträchtigen würden. Darüber hinaus wird weitestgehend vermieden, daß kleine Gasbläschen an den Leitfähigkeitselektroden durch Anhaften und Abreißen starke Meßwertschwankungen (Rauschen) verursachen, was ebenfalls das Signal beeinflussen würde.

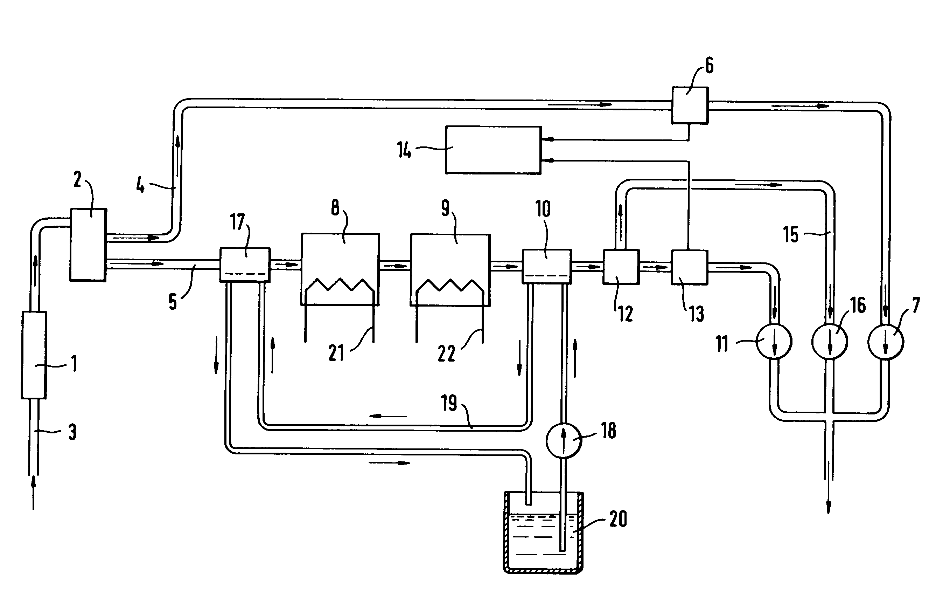

## Patentansprüche

1. Verfahren zum Überwachen von Wasser, insbesondere von Kesselspeisewasser auf organische Verunreinigungen durch Messen der Leitfähigkeit, dadurch gekennzeichnet, daß man von einem Probenwasserstrom einen Teilstrom abzweigt und die Leitfähigkeit mißt, den anderen Teilstrom bei 300-500° C verdampft, den Dampf thermisch bei Temperaturen von 800-1000° C behandelt, den behandelten Dampf kondensiert, die Leitfähigkeit des Kondensats mißt und die Differenz der gemessenen Leitfähigkeiten bildet.

2. Verfahren nach Anspruch 1, dadurch gekennzeichnet, daß man nach dem Kondensieren des Dampfes vorhandenes Gas abtrennt und Kondensat und Gas getrennt fördert.

3. Verfahren nach Anspruch 1 oder 2, dadurch gekennzeichnet, daß man den Probenwasserstrom vor dem Teilen durch einen lonenaustauscher leitet.

4. Vorrichtung zum Durchführen des Verfahrens nach Anspruch 1, dadurch gekennzeichnet, daß einem Probenwasserstromteiler (2) zwei Leitfähigkeitsmeßzellen (6, 13) nachgeschaltet sind, wobei zwischen Probenwasserstromteiler (2) und einer der Leitfähigkeitsmeßzellen (6, 13) in Richtung Meßzelle ein Verdampfer (8), ein Reaktor (9) und ein Kondensator (10) in Reihenschaltung angeordnet sind, und die Meßzellen (6, 13) mit einer elektronischen Meßwertverarbeitungseinrichtung (14) verbunden sind.

5. Vorrichtung nach Anspruch 4, dadurch gekennzeichnet, daß dem Kondensator (10) ein Gasabscheider (12) nachgeschaltet ist.

6. Vorrichtung nach Anspruch 4 oder 5, dadurch gekennzeichnet, daß dem Verdampfer (8) eine Dampfsperre (17) vorgeschaltet ist.

7. Vorrichtung nach Anspruch 4, dadurch gekennzeichnet, daß dem Probenwasserstromteiler (2) ein lonenaustauscher (1) vorgeschaltet ist.

8. Vorrichtung nach Anspruch 4, dadurch gekennzeichnet, daß den Meßzellen (6, 13) Fördereinrichtungen (7, 11) für die Probenwasserteilströme nachgeschaltet sind.

## Claims

1. A process for monitoring water, in particular boiler feed water, for organic impurities by measuring the conductivity, which comprises diverting a part stream from a sample water stream and measuring the conductivity, evaporating the other part stream at 300-500°C, treating the steam thermally at temperatures of 800-1000°C, condensing the treated steam, measuring the conductivity of the condensate and taking the difference between the measured conductivities.

2. The process as claimed in claim 1, wherein after condensing the steam any gas present is separated off and condensate and gas are fed separately.

3. The process as claimed in claim 1 or 2, wherein the sample water stream is passed through an ion exchanger before division.

4. A device for carrying out the process as claimed in claim 1, wherein two conductivity measuring cells (6, 13) are connected at the outlet of a sample water stream divider (2), an evaporator (8), a reactor (9) and a condenser (10) being arranged in series connection between the sample water stream divider (2) and one of the conductivity measuring cells (6, 13) in the measuring cell direction, and the measuring cells (6, 13) being connected to an electronic measurement processing arrangement (14).

5. The device as claimed in claim 4, wherein a gas separator (12) is connected at the outlet of the condenser (10).

6. The device as claimed in claim 4 or 5, wherein a steam baffle (17) is connected in series with the evaporator (8).

7. The device as claimed in claim 4, wherein an ion exchanger (1) is connected in series with the sample water stream divider (2).

8. The device as claimed in claim 4, wherein feed arrangements (7, 11) for the sample water part streams are connected at the outlet of the measuring cells (6, 13).

## Revendications

1. Méthode pour le contrôle de contaminations organiques dans l'eau, notamment l'eau d'approvisionnement de chaudières, par la mesure de la conductivité, caractérisée en ce que l'on fait dériver d'un flux d'eau à analyser un flux partiel dont on mesure la conductivité et que l'on fait évaporer l'autre flux partiel à 300 à 500° C, que l'on fait subir à la vapeur un traitement thermique à 800 à 1000° C, que l'on condense la vapeur traitée, que l'on mesure la conductivité du condensat et que l'on établit la différence des conductivités mesurées.

2. Méthode selon la revendication 1, caractérisée en ce que après la condensation de la vapeur, l'on sépare le gaz présent et que l'on transporte séparément le condensat et le gaz.

3. Méthode selon l'une des revendications 1 ou 2, caractérisée en ce que l'on fait passer le flux d'eau à analyser avant la séparation à travers un échangeur d'ions.

4. Dispositif pour l'exécution de la méthode selon la revendication 1, caractérisé en ce que deux cellules de mesure de la conductivité (6, 13) sont placées en aval d'un séparateur du flux d'eau à analyser (2), un évaporateur (8), un réacteur (9) et un condensateur (10) étant agencés en ligne entre le séparateur du flux d'eau à analyser (2) et l'une des cellules de mesure de la conductivité (6, 13), et que les cellules de mesure de la conductivité (6, 13) sont connectées à un dispositif électronique de traitement des valeurs mesurées (14).

5. Dispositif selon la revendication 4, caractérisé en ce que un séparateur de gaz (12) est situé en aval du condensateur (10).

6. Dispositif selon l'une des revendications 4 ou 5, caractérisé en ce que un piège à vapeur (17) est situé en amont de l'évaporateur (8).

7. Dispositif selon la revendication 4, caractérisé en ce que un échangeur d'ions (1) est situé en amont du séparateur du flux d'eau à analyser (2).

8. Dispositif selon la revendication 4, caractérisé en ce que des dispositifs de transport (7, 11) des flux partiels d'eau à analyser sont agencés en aval des cellules de mesure de la conductivité (6, 13).
